# EUROPEAN PATENT APPLICATION

(11) **EP 4 094 786 A1**
(43) Date of publication of application: **30.11.2022**
(21) Application number: 21743752.4
(22) Date of filing: 19.01.2021
(51) Int. Cl.: A61M 5/142, A61M 5/168, A61M 39/22

(54) **CONTRAST MEDIUM INJECTION DEVICE AND INJECTION LINE KIT**

(30) Priority: 20.01.2020 JP 2020006896
(71) Applicant: DVX Inc., Toshima-Ku Tokyo 171-0033 (JP)
(72) Inventor: FUJIOKA Tetsuya, Tokyo 171-0033 (JP); OHSHITA Kunio, Tokyo 171-0033 (JP)
(74) Representative: V.O.
(86) International application number: PCT/JP2021/001606
(87) International publication number: WO 2021/149664

(57) **Abstract**

Provided is a contrast medium injection device which easily removes air bubbles in a drug solution in a passage, uses inexpensive tubing, and is capable of measuring blood pressure even in the case of an external power outage.

Disclosed is a contrast medium injection device 1 having one or more liquid injection passages and an opening/blocking means provided to the liquid injection passages, wherein: the opening/blocking means has a generally cylindrical space 80 formed therein, and a spherical body 82 positioned inside the generally cylindrical space 80 so as to be capable of moving between a first end section 74 and a second end section 75 which faces the first end section 74; and the spherical body 82 blocks the liquid injection passages when adhered to the first end section 74 and opens the liquid injection passages when the state of being adhered to the first end section 74 is released.

## Description

### [Technical Field]

The present disclosure relates to a contrast medium injection system and an injection line kit which are used in injecting a contrast medium and other drug solutions into a patient in angiography.

### [Background Art]

In recent years, angiography has been used in medical sites, in which X-rays are repeatedly taken while injecting a contrast medium from a catheter into blood vessels using a contrast medium injection device to evaluate the morphology of the blood vessels and the state of blood flow. In contrast medium injection devices in angiography, flow paths are automatically switched by valves on the flow paths in accordance with operations such as filling a syringe with a contrast medium, monitoring the blood pressure of a patient, injecting the contrast medium into the patient, protecting a pressure transducer during the injection of the contrast medium, or injecting saline.

Such switching of flow paths is disclosed in Patent Document 1.

### [Prior Art Document]

### [Patent Document]

Patent Document 1: WO2014/020692 A1

### [Summary of the Invention]

### [Problems to be Solved by the Invention]

However, in the above-described prior art, opening and blocking operations required for switching flow paths are conducted by opening and squeezing the tubing that forms the flow paths with a clamp member, causing the following problems.

Specifically, air bubbles in a drug solution in a flow path should be removed before injecting the drug solution into a patient, and such removal often require effort. Since flow paths are blocked by squeezing the tubes that form the flow paths, the tubes to be used must have flexibility and pressure resistance. This necessitates the use of expensive tubes in which pressure-resistant fiber is woven. Although the patient's blood pressure should be able to be measured at all times, conventional contrast medium injection devices cannot measure blood pressure if external power supply is disconnected and stopped when the tube of the blood pressure circuit is squeezed. In addition, if external power supply is disconnected, the drug solution or body fluids flow back, contaminating the contrast medium in the syringe and making it impossible to reuse the contrast medium.

Accordingly, the present disclosure provides a contrast medium injection system that enables easy removal of air bubbles that are generated in a drug solution in a flow path during injection of the drug solution into a patient, use of inexpensive tubes, and measurement of the blood pressure of the patient and keeping the cleanliness of the contrast medium in a syringe in any situation including unexpected power outage of the contrast medium injection system during the injection.

### [Means for Solving the Problems]

In order to solve the above-mentioned problems, the present disclosure is understood from the following:
(1) According to a first aspect of the present disclosure, a contrast medium injection system includes: at least one fluid injection flow path; and an opening and blocking unit provided in the fluid injection flow path. The opening and blocking unit includes: a generally cylindrical space provided therein; and a spherical body that is disposed so as to be movable between a first end portion and a second end portion opposite to the first end portion in the generally cylindrical space. The spherical body blocks the fluid injection flow path when the spherical body is in close contact with the first end portion, and the fluid injection flow path is opened when a state in which the spherical body is in close contact with the first end portion is released.
(2) In the configuration (1), the spherical body maintains a state in which the fluid injection flow path is opened when the spherical body is in close contact with the second end portion.
(3) In the configuration (1) or (2), the second end portion is a tubular body that has a through hole in an axial direction therein and a through channel that extends from the through hole to an outer circumferential surface of the tubular body.
(4) In any one of the configurations (1) to (3), the opening and blocking unit includes a container that has a transparent portion, and the generally cylindrical space is formed in the container.
(5) In any one of the configurations (1) to (4), the contrast medium injection system includes: a magnet disposed outside of the generally cylindrical space; and a magnet moving unit that moves the magnet between a position opposed to the first end portion in the generally cylindrical space and a position opposed to the second end portion in the generally cylindrical space. The spherical body is a magnetic body. The spherical body moves between the first end portion and the second end portion in the generally cylindrical space in accordance with movement of the magnet between the position opposed to the first end portion in the generally cylindrical space and the position opposed to the second end portion in the generally cylindrical space which is caused by the magnet moving unit.
(6) In any one of the configurations (1) to (5), the opening and blocking unit includes a spherical body detecting unit that is configured to detect the spherical body.
(7) In the configuration (6), the spherical body detecting unit detects that the spherical body is at a position where the spherical body is in close contact with the first end portion.
(8) In the configuration (6) or (7), the spherical body detecting unit is an infrared sensor.
(9) According to a second aspect of the present disclosure, a contrast medium injection system includes: a fluid injection flow path; a liquid pressure measuring unit installed in the fluid injection flow path; and an opening and blocking unit that is installed in the fluid injection flow path and is configured to open and block the fluid injection flow path. The opening and blocking unit opens the fluid injection flow path when power supply to the contrast medium injection system is shut off.
(10) In the configuration (9), the fluid injection flow path includes a plurality of fluid injection flow paths. A second fluid injection flow path that is not the fluid injection flow path in which the liquid pressure measuring unit is installed has a second opening and blocking unit configured to open and block the second fluid injection flow path. The second opening and blocking unit blocks the second fluid injection flow path when power supply to the contrast medium injection system is shut off.
(11) According to a third aspect of the present disclosure, an injection line kit includes: at least one fluid injection flow path; an opening and blocking unit that is provided in the fluid injection flow path and is configured to open and block the fluid injection flow path; and a connector provided upstream of the opening and blocking unit. A downstream-side fluid injection flow path including the opening and blocking unit of the fluid injection flow path is configured to be attachable and detachable via the connector with respect to an upstream-side fluid injection flow path of the fluid injection flow path.
(12) In the configuration (11), the injection line kit includes a bridge and the downstream-side fluid injection flow path held by the bridge. The opening and blocking unit in the downstream-side fluid injection flow path includes: a generally cylindrical space therein; and a spherical body that is disposed so as to be movable between a first end portion and a second end portion opposite to the first end portion in the generally cylindrical space. The spherical body blocks the downstream-side fluid injection flow path when the spherical body is in close contact with the first end portion, and the downstream-side fluid injection flow path is opened when a state in which the spherical body is in close contact with the first end portion is released.
(13) In the configuration (12), the spherical body maintains a state in which the downstream-side fluid injection flow path is opened when the spherical body is in close contact with the second end portion.
(14) In the configuration (12) or (13), the second end portion is a tubular body that has a through hole in an axial direction therein and a through channel that extends from the through hole to an outer circumferential surface of the tubular body.
(15) In any one of the configurations (12) to (14), the opening and blocking unit includes a container that has a transparent portion, and the generally cylindrical space is formed in the container.

### [Effects of the Invention]

According to the present disclosure, it is possible to provide a contrast medium injection system that enables easy removal of air bubbles that are generated in a drug solution in a flow path during injection of the drug solution into a patient, use of inexpensive tubes, and measurement of the blood pressure of the patient and keeping the cleanliness of the contrast medium in a syringe in any situation including unexpected power outage of the contrast medium injection system during the injection.

### [Brief Description of the Drawings]

[FIG. 1] FIG. 1 is a diagram that illustrates an overall contrast medium injection system according to an embodiment of the present disclosure.
[FIG. 2] FIG. 2 is a pair of diagrams for explaining an injection line kit according to an embodiment of the present disclosure, in which (a) is an overall perspective view, and (b) is a perspective view as viewed from a side where connectors are.
[FIG. 3] FIG. 3 is a diagram for explaining attachment and detachment of the injection line kit with respect to the contrast medium injection system according to an embodiment of the present disclosure.
[FIG. 4] FIG. 4 is a collection of diagrams for explaining the attachment and detachment of the injection line kit with respect to the contrast medium injection system according to an embodiment of the present disclosure, in which (a) is a pair of diagrams for explaining a case in which the injection line kit is in a first set position, and (b) is a pair of diagrams for explaining a case in which the injection line kit is in a second set position.
[FIG. 5] FIG. 5 is a collection of diagrams for explaining an opening and blocking unit of the contrast medium injection system according to an embodiment of the present disclosure, in which (a) is a diagram for explaining a case in which a spherical body is in close contact with a first end portion, (b) is a diagram for explaining a case in which the spherical body is in close contact with a second end portion, and (c) is an enlarged view of the second end portion.
[FIG. 6] FIG. 6 is a pair of diagrams for explaining an operation of the opening and blocking unit of the contrast medium injection system according to an embodiment of the disclosure, in which (a) is a diagram for explaining an operation of the spherical body, and (b) is a diagram for explaining an operation of the overall opening and blocking unit.
[FIG. 7] FIG. 7 is a pair of diagrams for explaining prevention of backflow of a contrast medium in the injection line kit according to an embodiment of the present disclosure, in which (a) is a perspective view of the injection line kit, and (b) is a side view of the injection line kit.

### [Modes for Carrying Out the Invention]

Hereinafter, embodiments of the present disclosure will be described in detail with reference to the attached drawings.

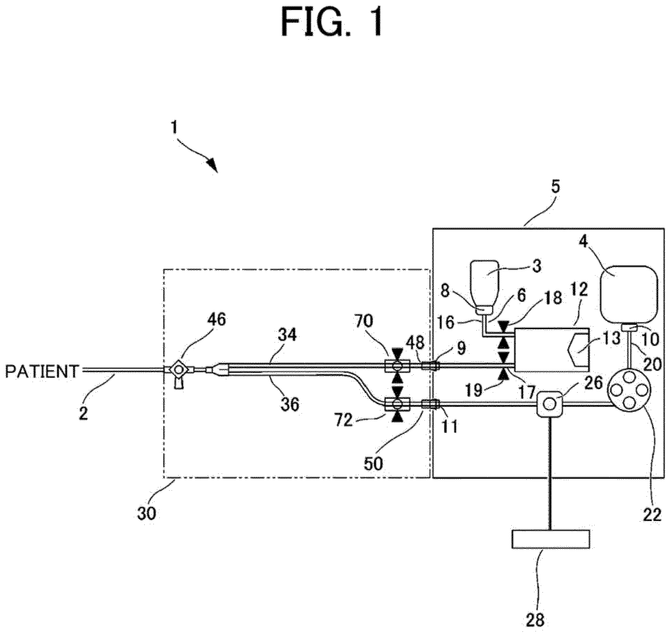

FIG. 1 is a diagram that illustrates an overall contrast medium injection system 1 according to an embodiment of the present disclosure.

As shown in FIG. 1, the contrast medium injection system 1 includes a contrast medium injection system body 5 and an injection line kit 30 and is coupled with a patient via a catheter 2. The contrast medium injection system 1 includes two systems of fluid injection flow paths: a contrast medium injection flow path 6 for injecting a contrast medium; and a saline injection flow path 20 which is filled with saline for measurement of the blood pressure of the patient.

In the description of the present embodiment, the side close to a contrast medium bottle 3 is defined to be the upstream of the contrast medium injection flow path 6, and the side close to the catheter 2 is defined to be the downstream thereof, unless otherwise specified. Similarly, the side close to a saline pack 4 is defined to be the upstream of the saline injection flow path 20, and the side close to the catheter 2 is defined to be the downstream thereof.

The contrast medium injection flow path 6 is provided with a contrast medium bottle connecting connector 8 on one end. The contrast medium bottle 3 is installed on the contrast medium bottle connecting connector 8 such that the contrast medium can be injected into the contrast medium injection system 1.

On the downstream of the contrast medium bottle connecting connector 8, a syringe 12 is provided. The syringe 12 is temporarily filled with the contrast medium and expels the contrast medium toward its downstream. Inside the syringe 12, a wiper 13 is provided. On a first connecting flow path 16 that connects the contrast medium bottle connecting connector 8 and the syringe 12 by means of a tube, a pinch valve 18 is provided, so that the flow path can be opened and blocked.

On the other end of the contrast medium injection flow path 6, a contrast medium injection connector 9 is provided. On a second connecting flow path 17 that connects the syringe 12 and the contrast medium injection connector 9 by means of a tube, a pinch valve 19 is provided, so that the flow path can be opened and blocked.

The saline injection flow path 20 is provided with a saline pack connecting connector 10 on one end. The saline pack 4 is installed on the saline pack connecting connector 10 such that the saline can be injected into the contrast medium injection system 1.

On the downstream of the saline pack connecting connector 10, a pump 22 is provided. The pump 22 delivers the saline supplied from the saline pack 4 toward its downstream. The pump 22 is often a roller pump which is suitable for delivering a drug solution in a tube, but it is not limited thereto.

On the other end of the saline injection flow path 20, a saline injection connector 11 is provided. Between the pump 22 and the saline injection connector 11, a blood pressure transducer 26 is installed as a liquid pressure measuring unit. The patient's blood pressure waves are detected and transformed by the blood pressure transducer 26 so as to be checked on a blood pressure monitor 28 which is an external device coupled to the blood pressure transducer 26.

As for connectors for coupling the contrast medium injection system body 5 with the injection line kit 30, a contrast medium injection connector 9 and a saline injection connector 11, which are first connectors provided on the contrast medium injection system body 5, are engaged with a contrast medium injection line receiving connector 48 and a saline injection line receiving connector 50, which are second connectors provided on the injection line kit 30, thereby coupling the contrast medium injection system body 5 and the injection line kit 30 together.

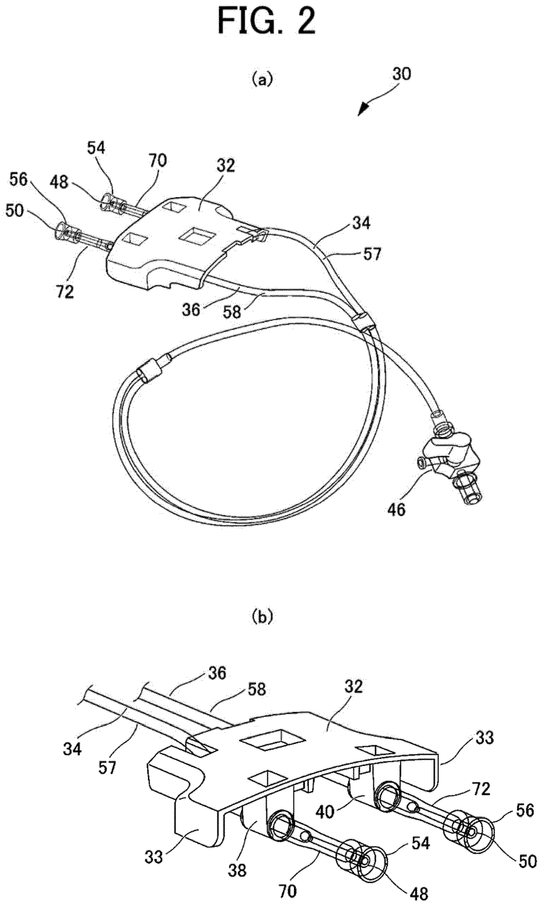

FIG. 2 is a pair of diagrams for explaining the injection line kit 30 according to the embodiment of the present disclosure, in which (a) is an overall perspective view, and (b) is a perspective view as viewed from the side where the connectors are.

The Injection line kit 30 will be described with reference to FIG. 2.

In the contrast medium injection system 1, the contrast medium injection system body 5 is provided on the upstream side, which is the side close to the contrast medium bottle 3 and the saline pack 4. The contrast medium injection system body 5 includes upstream-side flow paths. The injection line kit 30 is provided on the side close to the catheter 2 and includes downstream-side flow paths.

In the injection line kit 30, a contrast medium injection line 34 and a saline injection line 36, which are patient connection flow paths to be coupled to the catheter 2 of a patient, are respectively held in a contrast medium injection line fixing section 38 and a saline injection line fixing section 40 in a bridge 32 so as to be substantially parallel to each other. The bridge 32 is formed with a grip section 33 for an operator to hold when handling the injection line kit 30. According to the present embodiment, the grip section 33 is provided as surfaces on the sides of the bridge 32, the surfaces being substantially parallel to each other, such that an operator can hold the bridge 32 between two fingers of his/her hand. However, it is not limited thereto, and the grip section 33 may be a handle, a convex knob, or the like. The grip section 33 may be integrally provided on the bridge 32 by resin molding or may be a separate component to be fixed to the bridge 32 by a screw or the like. Further, the grip section 33 may be provided with concave and convex portions formed on its surface, a rubber sheet adhered on its surface or the like to prevent slippage.

The contrast medium injection line 34 includes a second tube 57 and a second opening and blocking unit 70 which are coupled to each other. The saline injection line 36 includes a first tube 58 and a first opening and blocking unit 72 which are coupled to each other. The second opening and blocking unit 70 and the first opening and blocking unit 72 are provided with the contrast medium injection line receiving connector 48 and the saline injection line receiving connector 50, respectively, on the opposite side of the side to connect with the second tube 57 and the first tube 58. On the outer surfaces of the contrast medium injection line receiving connector 48 and the saline injection line receiving connector 50, a guide cover 54 and a guide cover 56 in a tapered shape are respectively provided. The guide cover 54 and the guide cover 56 each have an inner diameter larger than the outer diameter of the contrast medium injection connector 9 and the saline injection connector 11 that are mating connectors of the contrast medium injection line receiving connector 48 and the saline injection line receiving connector 50, respectively. The guide cover 54 and the guide cover 56 begin to become bigger in diameter in the vicinity of the tip ends of the contrast medium injection line receiving connector 48 and the saline injection line receiving connector 50 to gradually expand toward the tip ends of the guide cover 54 and the guide cover 56. Thus, the tip ends of the guide cover 54 and the guide cover 56 are positioned so as to protrude relative to the tip ends of the contrast medium injection line receiving connector 48 and the saline injection line receiving connector 50. The contrast medium injection line receiving connector 48 and the saline injection line receiving connector 50 are oriented in the same direction to be substantially in parallel with each other and fixed with their tip ends aligned. On the other end of the second tube 57 and the first tube 58, there is a three-way stopcock 46, via which the second tube 57 and the first tube 58 are coupled with the catheter 2 of the patient.

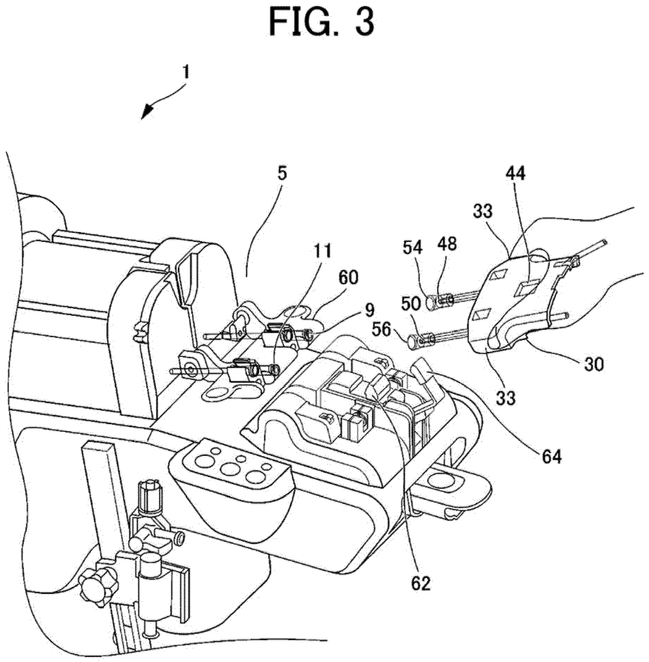

FIG. 3 is a diagram for explaining attachment and detachment of the injection line kit 30 with respect to the contrast medium injection system 1 according to the embodiment of the present disclosure.

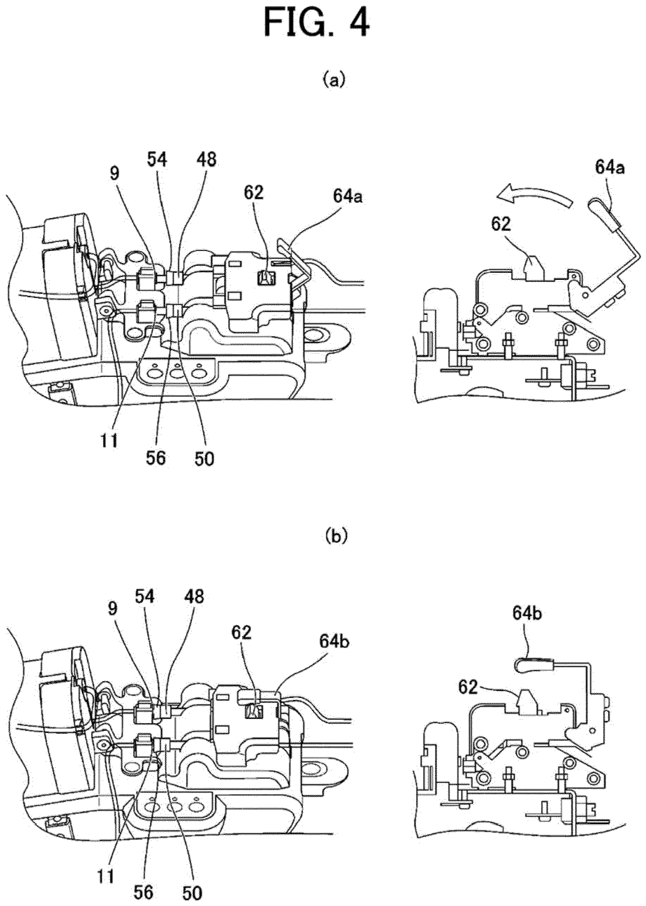

FIG. 4 is a collection of diagrams for explaining the attachment and detachment of the injection line kit 30 with respect to the contrast medium injection system 1 according to the embodiment of the present disclosure, in which (a) is a pair of diagrams for explaining a case in which the injection line kit 30 is in a first set position, and (b) is a pair of diagrams for explaining a case in which the injection line kit 30 is in a second set position.

According to the present embodiment, the injection line kit 30 is configured to be attachable and detachable with respect to the contrast medium injection system body 5.

The attachment of the injection line kit 30 to the contrast medium injection system body 5 will described with reference to FIG. 3 and FIG. 4. As shown in FIG. 3, in the contrast medium injection system 1, the contrast medium injection connector 9 and the saline injection connector 11 are fixed on a plate 60 of the contrast medium injection system body 5 so as to be oriented in the same direction to be substantially in parallel with each other and fixed with their tip ends aligned. The inner diameters of the tip ends of the guide cover 54 and the guide cover 56 of the injection line kit 30 are larger than the outer diameters of the tip ends of the contrast medium injection connector 9 and the saline injection connector 11, and the tip ends of the guide cover 54 and the guide cover 56 are positioned so as to protrude relative to the tip ends of the contrast medium injection line receiving connector 48 and the saline injection line receiving connector 50. Therefore, when inner diameter portions of the tip ends of the guide cover 54 and the guide cover 56 are placed and pushed onto the outer profiles of the tip ends of the contrast medium injection connector 9 and saline injection connector 11, the contrast medium injection line receiving connector 48 and the saline injection line receiving connector 50 are guided by the tapers of the guide cover 54 and the guide cover 56 into loose engagement with the contrast medium injection connector 9 and the saline injection connector 11, respectively.

In this state, when the injection line kit 30 and the plate 60 are pressed down together, an injection line kit lock 62 provided on the contrast medium injection system body 5 is locked in a lock mating opening 44 formed in the bridge 32 of the injection line kit 30, so that the injection line kit 30 is set in the first set position, as illustrated in FIG. 4 (a). Further, moving a lever 64 from a first position 64a to a second position 64b causes the injection line kit 30 to slide toward the contrast medium injection connector 9 and the saline injection connector 11, so that the contrast medium injection line receiving connector 48 and the saline injection line receiving connector 50 in the loose engagement come into tight engagement with the contrast medium injection connector 9 and the saline injection connector 11, respectively, to establish a secure connection. In this state, the injection line kit 30 is completely fixed to the contrast medium injection system body 5 in the second set position as illustrated in FIG. 4 (b). The lever 64 is configured to slide the injection line kit 30 and fix it in place by using the principle of leverage, so that an operator can easily operate it with a weak force and the connectors can be securely coupled together. Thus, the flow path from the contrast medium bottle 3 to the three-way stopcock 46 at the distal end is established in the contrast medium injection flow path 6, and the flow path from the saline pack 4 to the three-way stopcock 46 at the distal end is established in the saline injection flow path 20, so that the respective drug solutions can be injected into a patient via the catheter 2 coupled to the three-way stopcock 46, and the patient's blood pressure can be measured.

Next, the detachment of the injection line kit 30 will be described. Once a predetermined angiographic examination is complete, the three-way stopcock 46 is disconnected from the catheter 2. Returning the lever 64 from the second position 64b to the first position 64a moves the injection line kit 30 that is in the second set position in a direction away from the contrast medium injection connector 9 and the saline injection connector 11 to return it to the first set position where the connectors are loosely engaged with each other. In this state, the injection line kit lock 62 is unlocked, and the injection line kit 30 and the plate 60 are pushed up together. The injection line kit 30 is then detached in an obliquely upward direction. The operator can easily detach the injection line kit 30 with a weak force since the contrast medium injection line receiving connector 48 and the saline injection line receiving connector 50 are only in loose engagement with the contrast medium injection connector 9 and the saline injection connector 11.

According to the present embodiment, the contrast medium injection connector 9 and the saline injection connector 11 are loosely engaged with the contrast medium injection line receiving connector 48 and the saline injection line receiving connector 50 respectively in the first set position, but it is not limited thereto. These connectors may not be in engagement with each other in the first set position and may be brought into engagement during the process of moving to the second set position and fully engaged with each other upon reaching the second set position. Also in this case, the tapers of the guide cover 54 and the guide cover 56 of the contrast medium injection line receiving connector 48 and the saline injection line receiving connector 50, respectively, similarly serve as guides for the connectors to engage together. Alternatively, the injection line kit lock 62 may be locked and the injection line kit 30 may be fixed in the first position when an operator sets the injection line kit 30 in the first set position. In the detachment of the injection line kit 30 in this case, since the connectors are already out of engagement in the first set position, the injection line kit 30 can be detached only by unlocking the injection line kit lock 62.

In the above-described attachment and detachment operations of the injection line kit 30 with respect to the contrast medium injection system body 5, the operator can hold the grip section 33 provided on the bridge 32 of the injection line kit 30 between his/her two fingers while handling the injection line kit 30 and operate the lever 64 to attach and detach the injection line kit 30 with respect with the contrast medium injection system body 5. Thus, the operator can conduct the operations without touching the contrast medium injection line receiving connector 48 and the saline injection line receiving connector 50 of the injection line kit 30 as well as the contrast medium injection connector 9 and the saline injection connector 11 of the contrast medium injection system body 5 at all, so that the cleanliness of the flow paths including these connectors can be maintained.

Furthermore, since the contrast medium injection line receiving connector 48 and the saline injection line receiving connector 50 of the injection line kit 30 including their tip ends are entirely covered with the guide cover 54 and the guide cover 56 respectively, the operator is prevented from inadvertently touching the contrast medium injection line receiving connector 48 and the saline injection line receiving connector 50 in normal handling such as setting and detaching of the injection line kit 30 with respect with the contrast medium injection system body 5, so that the cleanliness of these connectors can be maintained.

An operation of filling the syringe 12 with a contrast medium will be described with reference to FIG. 1. The pinch valve 18 on the first connecting flow path 16 is opened, and the pinch valve 19 on the second connecting flow path 17 is blocked. The wiper 13 of the syringe 12 is retracted to suck a contrast medium from the contrast medium bottle 3 to fill the syringe 12 with the contrast medium.

Priming of the contrast medium into the contrast medium injection flow path 6 will be described. The pinch valve 18 is blocked, and the pinch valve 19 and the second opening and blocking unit 70 are opened. Next, the wiper 13 of the syringe 12 is advanced, and the injection is continued until the contrast medium injection flow path 6 is filled with the contrast medium from the syringe 12. When the contrast medium injection flow path 6 is filled with the contrast medium, the wiper 13 is stopped and the pinch valve 19 is blocked.

Priming of saline into the saline injection flow path 20 will be described. The first opening and blocking unit 72 is opened, and the pump 22 is driven to inject the saline until the saline injection flow path 20 is filled with the saline.

Monitoring of blood pressure will be described. The saline injection flow path 20 is filled with the saline, and the three-way stopcock 46 is coupled to the catheter 2 that is indwelling in the patient's blood vessel. The patient's blood pressure waves are transmitted through the saline to the blood pressure transducer 26 and displayed on the blood pressure monitor 28, which is a coupled external device.

An injection operation of the contrast medium into a patient will be described. The first opening and blocking unit 72 in the saline injection flow path 20 is blocked to protect the blood pressure transducer 26 from excessive pressure. The second opening and blocking unit 70 in the contrast medium injection flow path 6 is opened, and the wiper 13 of the syringe 12 is advanced to inject the contrast medium. Upon completing the injection, the second opening and blocking unit 70 is blocked, and the first opening and blocking unit 72 is opened to start the monitoring of the blood pressure.

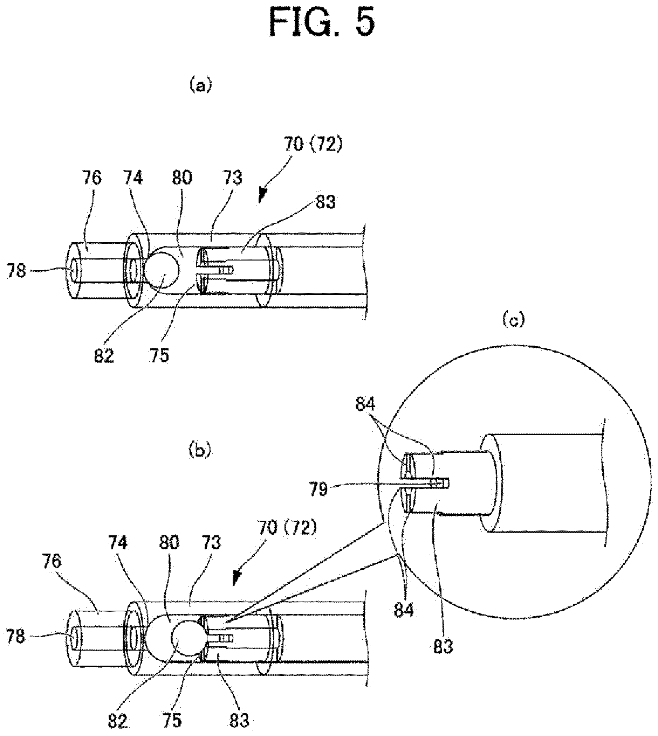

FIG. 5 is a collection of diagrams for explaining the opening and blocking units of the contrast medium injection system 1 according to the embodiment of the present disclosure, in which (a) is a diagram for explaining a case in which the spherical body 82 is in close contact with a first end portion 74, (b) is a diagram for explaining a case in which the spherical body 82 is in close contact with a second end portion 75, (c) is an enlarged view of the second end portion 75.

The second opening and blocking unit 70 and the first opening and blocking unit 72 will be described with reference to FIG. 5. The second opening and blocking unit 70 and the first opening and blocking unit 72 each include a generally cylindrical space 80 in a container 73. In the generally cylindrical space 80, the spherical body 82, which is a magnetic body, is disposed so as to movable between the first end portion 74 and the second end portion 75.

As for the first end portion 74 of the generally cylindrical space 80, a cylindrical tubular body 76 is formed integrally with the container 73. A flow path hole 78 which has a diameter smaller than the diameter of the spherical body 82 penetrates through a central portion of the cylindrical tubular body 76 to form a flow path. As illustrated in FIG. 5 (a), when the spherical body 82 comes into close contact with the first end portion 74 of the cylindrical tubular body 76, the flow path hole 78 is closed by the spherical body 82, so that the flow path is blocked.

As for the second end portion 75 of the generally cylindrical space 80, a cylindrical tubular body 83 is press-fitted into the container 73. A flow path hole 79 penetrates through a central portion of the cylindrical tubular body 83 in the axial direction to form a through hole. The flow path hole 79 has a diameter smaller than the diameter of the spherical body 82 at the second end portion 75. U-shaped slits 84 are formed so as to extend from the second end portion 75 of the cylindrical tubular body 83 and to communicate with the flow path hole 79. As illustrated in FIG. 5 (b), although the flow path hole 79 is closed by the spherical body 82 when the spherical body 82 comes into close contact with the second end portion 75 of the cylindrical tubular body 83, the flow path is kept open and is not blocked since the drug solution flows through the slits 84. When the state in which the spherical body 82 is in close contact with the first end portion 74 of the cylindrical tubular body 76 is released and the spherical body 82 is at a position between the first end portion 74 and the second end portion 75 with no contact with the ends 74, 75, the flow path is kept open and is not blocked since there is a clearance between the inner surface of the container 73 which has the generally cylindrical space 80 therein and the outer surface of the spherical body 82.

According to the present embodiment, the U-shaped slits 84 are provided. However, the same result can be obtained by a pass-through hole that is provided at an appropriate position away from the second end portion 75 of the cylindrical tubular body 83 so as to penetrate from the flow path hole 79 to the outer circumferential surface of the cylindrical tubular body 83. Through channels such as the slits and the pass-through hole may be provided as many as appropriate, and the number thereof may be one or more, for example.

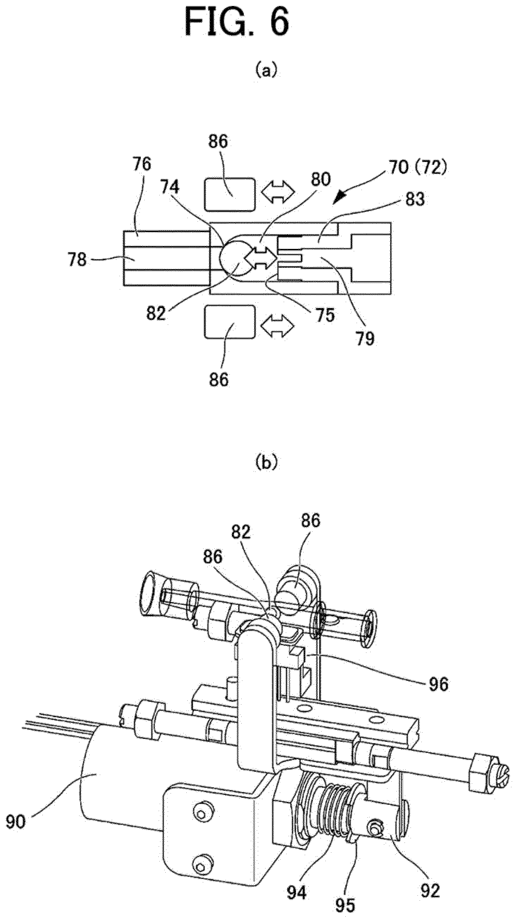

FIG. 6 is a pair of diagrams for explaining an operation of the opening and blocking unit of the contrast medium injection system 1 according to the embodiment of the disclosure, in which (a) is a diagram for explaining an operation of the spherical body 82, and (b) is a diagram for explaining an operation of the overall opening and blocking unit.

The operation of the second opening and blocking unit 70 and the first opening and blocking unit 72 will be described with reference to FIG. 6. As illustrated in FIG. 6 (a), magnets 86 are provided outside the second opening and blocking unit 70 and the first opening and blocking unit 72. The magnets 86 are moved by a magnet moving unit (not illustrated) driven by an electromagnetic solenoid 90 between a position opposed to the first end portion 74 of the cylindrical tubular body 76 of the second opening and blocking unit 70 and the first opening and blocking unit 72 and a position opposed to the second end portion 75 of the cylindrical tubular body 83. Accordingly, the spherical body 82, which is a magnetic body inside the second opening and blocking unit 70 and the first opening and blocking unit 72, is attracted by the magnetic force of the magnets 86 to move between the first end portion 74 of the cylindrical tubular body 76 and the second position 75 of the cylindrical tubular body 83. When the spherical body 82 is in close contact with the first end portion 74 of the cylindrical tubular body 76, the flow path is blocked, and when the spherical body 82 is close contact with the second end portion 75 of the cylindrical tubular body 83, the flow path is opened. According to the present embodiment, the spherical body 82 is a sphere, but it is not limited thereto. The same effect can be obtained by an ellipsoid, a columnar body or the like in relationship to the ends, for example.

As illustrated in FIG. 6 (b), outside the second opening and blocking unit 70 and the first opening and blocking unit 72, an infrared sensor 96 is provided as a spherical body detecting unit that detects that the spherical body 82 is at the position where the spherical body 82 is in close contact with the first end portion 74 of the cylindrical tubular body 76. Based on the detected data, energization of the electromagnetic solenoid 90 is controlled. According to the present embodiment, the infrared sensor 96 is used to detect the spherical body 82, but it is not limited thereto. Any visible light sensor or other sensors may be used as long as it is capable of detecting the spherical body 82. In addition, the position to be detected and the number of sensors provided are not limited to those according to the present embodiment. Further, the electromagnetic solenoid 90 is not limited to a plunger solenoid, and it may be a rotary solenoid. Alternatively, a motor may be used for driving the magnet moving unit.

In the second opening and blocking unit 70, the electromagnetic solenoid 90 includes a compression spring 94 which is held to the plunger 92 by a retaining ring 95. When the electromagnetic solenoid 90 is energized to attract the plunger 92, the spherical body 82 comes into close contact with the second end portion 75 of the cylindrical tubular body 83 to open the flow path, and when the energization is stopped, the spring 94 returns the plunger 92 back, so that the spherical body 82 comes into close contact with the first end portion 74 of the cylindrical tubular body 76 to block the flow path. Hence, in the contrast medium injection flow path 6, the flow path is kept blocked when power supply to the contrast medium injection system 1 is shut off. Thus, the backflow of the drug solution or body fluids to the syringe 12 is prevented even in the case of a power outage, so that the contrast medium filled in the syringe 12 can be kept clean, allowing the contrast medium to be used plural times.

On the other hand, also in the first opening and blocking unit 72, the electromagnetic solenoid 90 includes the compression spring 94 which is held to the plunger 92 by the retaining ring 95. When the electromagnetic solenoid 90 is energized to attract the plunger 92, the spherical body 82 comes into close contact with the first end portion 74 of the cylindrical tubular body 76 to block the flow path, and when the energization is stopped, the spring 94 returns the plunger 92 back, so that the spherical body 82 comes into close contact with the second end portion 75 of the cylindrical tubular body 83 to open the flow path. Hence, in the saline injection flow path 20, the flow path is kept opened when power supply to the contrast medium injection system 1 is shut off, thereby allowing the measurement of the patient's blood pressure even in a power outage.

In other words, when power supply to the contrast medium injection system 1 is shut off, the second opening and blocking unit 70 of the contrast medium injection flow path 6 is configured to be placed in the blocking state to block the flow path, and the first opening and blocking unit 72 of the saline injection flow path 20 is configured to be placed in the open state to open the flow path.

Next, an operation for removing air bubbles which is conducted by the second opening and blocking unit 70 and the first opening and blocking unit 72 will be described.

It is especially important to remove any air bubbles generated in the flow paths of the contrast medium injection flow path 6 and the saline injection flow path 20 to prevent the air bubbles from being accidentally injected into a patient for securing the patient's safety.

The container 73 of the second opening and blocking unit 70 and the first opening and blocking unit 72 has a transparent portion to allow visual checking from the outside whether there are any air bubbles. When air bubbles are found, a switch for removing air bubbles (not illustrated) is turned on. Accordingly, the energization of the electromagnetic solenoid 90 is repeatedly switched between on and off at a high speed, causing the spherical body 82 to continuously reciprocate in the generally cylindrical space 80 at a high speed in accordance with the movement of the magnets 86. This reciprocating motion generates complex liquid flows in the flow path, which peels off air bubbles attached on the sphere which are difficult to be removed at a low flow rate. The air bubbles are then discharged to the outside of the contrast medium injection system 1 through the three-way stopcock 46 by the flow in the flow path caused by the pump 22. In particular, complex flows that pass through the U-shaped slits 84 are generated in the vicinity of the second end portion 75 of the cylindrical tubular body 83, which effectively peels off and removes air bubbles attached in this vicinity. As for the transparent portion of the container 73, the entire container 73 or a part of the container 73 which is defined in the circumferential direction and along the flow path may be a transparent member such that the generation of air bubbles can be recognized from the outside. Further, the transparent material may be made of glass or a transparent thermoplastic resin such as polycarbonate, acrylic or the like.

According to the present embodiment, the second opening and blocking unit 70 and the first opening and blocking unit 72 are the same, but it is not limited thereto.

In the contrast medium injection line 34 and the saline injection line 36, the second opening and blocking unit 70 and the first opening and blocking unit 72 are coupled with the three-way stopcock 46 provided at the distal end using the second tube 57 and the first tube 58, respectively. Since the second opening and blocking unit 70 and the first opening and blocking unit 72 are configured to switch the opening and blocking of the flow path by changing the position of the spherical body 82, the tubing does not need to be squeezed for the switching. Hence, the second tube 57 and the first tube 58 may not be an expensive tube that has flexibility and pressure resistance imparted by pressure resistant fiber woven therein (e.g., a braided tube), and it becomes possible to use any inexpensive tube (e.g., a hard tube) of which material or hardness is not particularly limited.

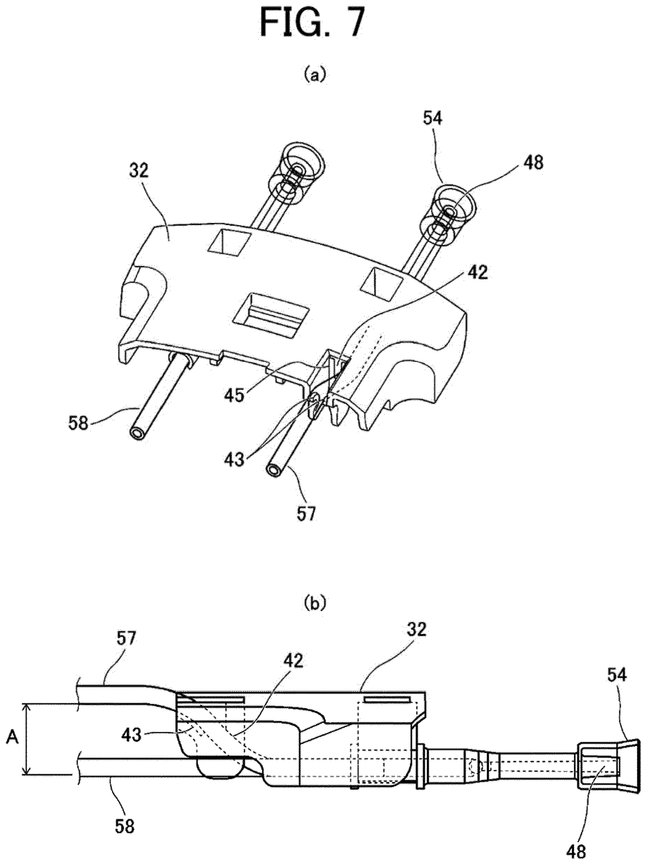

FIG. 7 is a pair of diagrams for explaining prevention of backflow of the contrast medium in the injection line kit 30 according to the embodiment of the present disclosure, in which (a) is a perspective view of the injection line kit 30, and (b) is a side view of the injection line kit 30.

Prevention of backflow of the contrast medium in the contrast medium injection line 34 of the injection line kit 30 will be described with reference to FIG. 7. The injection line kit 30 is discarded after each examination of a patient, but the contrast medium injection flow path 6 of the contrast medium injection system body 5 is used repeatedly in plural examinations. Hence, the patient's blood needs to be stopped at the patient side of the injection line kit 30. Generally, contrast media have a specific gravity heavier than that of blood, and blood tends to get on top of a contrast medium when the contrast medium and the blood are in a mixed state. Accordingly, when the upstream side of the contrast medium injection line 34 is at a position higher than the patient connection portion, replacement between the blood and the contrast medium starts in the tube and the blood flows upstream while being on top of the contrast medium and enters the contrast medium injection system body 5 from the injection line kit 30. In the case of the injection line kit according to the present disclosure, which is generally coupled with a device that is installed and operated at a position higher than a patient, the replacement between the blood and the contrast medium occurs due to the difference in the specific gravities. Although the second opening and blocking unit 70 always blocks the flowback except for during the injection operation, a contrast medium anti-flowback guide 42 is provided in the bridge 32 of the injection line kit 30 in order to further ensure the prevention of the flowback of the contrast medium mixed with the blood. The contrast medium anti-flowback guide 42 is a convex portion which extends upward approximately from the height of the contrast medium injection line receiving connector 48. The second tube 57 of the contrast medium injection line 34 is guided so as to extend along the contrast medium anti-flowback guide 42 to form a stepped portion having a step A at a middle portion of the second tube 57. Further, the bridge 32 is provided with, on its end on the opposite side of the contrast medium injection line receiving connector 48, a tube support section 43 which supports the second tube 57. According to the present embodiment, the tube support section 43 is provided such that a groove 45 having a width substantially equal to the outer diameter of the second tube 57 is formed on the end of the bridge 32 on the opposite side of the contrast medium injection line receiving connector 48; a protrusion is provided at the bottom of the groove 45 so as to protrude toward the inside of the groove 45; and the second tube 57 is supported by this protrusion and restricted in the width direction by the groove 45. However, the configuration is not limited thereto. The second tube 57 is supported by and placed on the tube support section 43 and cooperates with the contrast medium anti-flowback guide 42 to stably form the stepped portion having the step A. The thus formed stepped portion having the step A prevents the patient's blood from flowing down to the contrast medium injection line receiving connector 48, so that the patient's blood stops before the contrast medium injection line receiving connector 48.

Embodiments of the present disclosure have been described in detail above. However, the present disclosure is not limited to a particular embodiment, and various variations and modifications may be made within the scope of the claims. It is also possible to combine all or some of the components of the embodiments described above.

### [List of Reference Signs]

- 1: Contrast medium injection system
- 2: Catheter
- 3: Contrast medium bottle
- 4: Saline pack
- 5: Contrast medium injection system body
- 6: Contrast medium injection flow path
- 8: Contrast medium bottle connecting connector
- 9: Contrast medium injection connector
- 10: Saline pack connecting connector
- 11: Saline injection connector
- 12: Syringe
- 13: Wiper
- 14: Wiper drive unit
- 16: First connecting flow path
- 17: Second connecting flow path
- 18: Pinch valve
- 19: Pinch valve
- 20: Saline injection flow path
- 22: Pump
- 26: Blood pressure transducer
- 28: Blood pressure monitor
- 30: Injection line kit
- 32: Bridge
- 33: Grip section
- 34: Contrast medium injection line
- 36: Saline injection line
- 38: Contrast medium injection line fixing section
- 40: Saline injection line fixing section
- 42: Contrast medium anti-flowback guide
- 43: Tube support section
- 44: Lock mating opening
- 45: Groove
- 46: Three-way stopcock
- 48: Contrast medium injection line receiving connector
- 50: Saline injection line receiving connector
- 54: Guide cover
- 56: Guide cover
- 57: Second tube
- 58: First tube
- 60: Plate
- 62: Injection line kit lock (lock)
- 64: Lever
- 70: Second opening and blocking unit
- 72: First opening and blocking unit
- 73: Container
- 74: First end portion
- 75: Second end portion
- 76: Cylindrical tubular body
- 78: Flow path hole
- 79: Flow path hole
- 80: Generally cylindrical space
- 82: Spherical body
- 83: Cylindrical tubular body
- 84: Slit
- 86: Magnet
- 90: Electromagnetic solenoid
- 92: Plunger
- 94: Compression spring
- 95: Retaining ring
- 96: Infrared sensor

## Claims

1. A contrast medium injection system, comprising:
at least one fluid injection flow path; and
an opening and blocking unit provided in the fluid injection flow path, wherein
the opening and blocking unit comprises:
a generally cylindrical space provided therein; and
a spherical body that is disposed so as to be movable between a first end portion and a second end portion opposite to the first end portion in the generally cylindrical space, and
the spherical body blocks the fluid injection flow path when the spherical body is in close contact with the first end portion, and the fluid injection flow path is opened when a state in which the spherical body is in close contact with the first end portion is released.

2. The contrast medium injection system according to claim 1, wherein the spherical body maintains a state in which the fluid injection flow path is opened when the spherical body is in close contact with the second end portion.

3. The contrast medium injection system according to claim 1 or 2, wherein the second end portion is a tubular body that has a through hole in an axial direction therein and a through channel that extends from the through hole to an outer circumferential surface of the tubular body.

4. The contrast medium injection system according to any one of claims 1 to 3, wherein the opening and blocking unit includes a container that has a transparent portion, and the generally cylindrical space is formed in the container.

5. The contrast medium injection system according to any one of claims 1 to 4, comprising:
a magnet disposed outside of the generally cylindrical space; and
a magnet moving unit that moves the magnet between a position opposed to the first end portion in the generally cylindrical space and a position opposed to the second end portion in the generally cylindrical space, wherein
the spherical body is a magnetic body, and
the spherical body moves between the first end portion and the second end portion in the generally cylindrical space in accordance with movement of the magnet between the position opposed to the first end portion in the generally cylindrical space and the position opposed to the second end portion in the generally cylindrical space which is caused by the magnet moving unit.

6. The contrast medium injection system according to any one of claims 1 to 5, wherein the opening and blocking unit comprises a spherical body detecting unit that is configured to detect the spherical body.

7. The contrast medium injection system according to claim 6, wherein the spherical body detecting unit detects that the spherical body is at a position where the spherical body is in close contact with the first end portion.

8. The contrast medium injection system according to claim 6 or 7, wherein the spherical body detecting unit is an infrared sensor.

9. A contrast medium injection system, comprising:
a fluid injection flow path;
a liquid pressure measuring unit installed in the fluid injection flow path; and
an opening and blocking unit that is installed in the fluid injection flow path and is configured to open and block the fluid injection flow path, wherein
the opening and blocking unit opens the fluid injection flow path when power supply to the contrast medium injection system is shut off.

10. The contrast medium injection system according to claim 9, wherein
the fluid injection flow path includes a plurality of fluid injection flow paths,
a second fluid injection flow path that is not the fluid injection flow path in which the liquid pressure measuring unit is installed has a second opening and blocking unit configured to open and block the second fluid injection flow path, and
the second opening and blocking unit blocks the second fluid injection flow path when power supply to the contrast medium injection system is shut off.

11. An injection line kit, comprising:
at least one fluid injection flow path;
an opening and blocking unit that is provided in the fluid injection flow path and is configured to open and block the fluid injection flow path; and
a connector provided upstream of the opening and blocking unit, wherein
a downstream-side fluid injection flow path including the opening and blocking unit of the fluid injection flow path is configured to be attachable and detachable via the connector with respect to an upstream-side fluid injection flow path of the fluid injection flow path.

12. The injection line kit according to claim 11, comprising:
a bridge, and
the downstream-side fluid injection flow path held by the bridge, wherein
the opening and blocking unit in the downstream-side fluid injection flow path comprises:
a generally cylindrical space therein; and
a spherical body that is disposed so as to be movable between a first end portion and a second end portion opposite to the first end portion in the generally cylindrical space, and
the spherical body blocks the downstream-side fluid injection flow path when the spherical body is in close contact with the first end portion, and the downstream-side fluid injection flow path is opened when a state in which the spherical body is in close contact with the first end portion is released.

13. The injection line kit according to claim 12, wherein the spherical body maintains a state in which the downstream-side fluid injection flow path is opened when the spherical body is in close contact with the second end portion.

14. The injection line kit according to claim 12 or 13, wherein the second end portion is a tubular body that has a through hole in an axial direction therein and a through channel that extends from the through hole to an outer circumferential surface of the tubular body.

15. The injection line kit according to any one of claims 12 to 14, wherein the opening and blocking unit includes a container that has a transparent portion, and the generally cylindrical space is formed in the container.
